# EUROPEAN PATENT APPLICATION

(11) **EP 4 414 452 A1**
(43) Date of publication of application: **14.08.2024**
(21) Application number: 23382131.3
(22) Date of filing: 13.02.2023
(51) Int. Cl.: C12N 15/113, C12Q 1/68

(54) **DUAL-GUIDE RNA COMPOSITION FOR EXECUTING A SINGLE-GUIDE RNA CRISPR-ASSOCIATED SYSTEM**

(71) Applicant: Universidad de Granada, 18071 Granada (ES); Servicio Andaluz de Salud, 41071 Sevilla (ES)
(72) Inventor: MARTIN MOLINA, FRANCISCO, 18071 Granada (ES); AGUILAR GONZALEZ, ARACELI, 18071 Granada (ES); DÍAZ MOCHÓN, JUAN JOSÉ, 18071 Granada (ES); SÁNCHEZ MARTÍN, ROSARIO MARÍA, 18071 Granada (ES); MALDONADO PEREZ, NOELIA, 41071 Sevilla (ES)
(74) Representative: Hoffmann Eitle

(57) **Abstract**

The present invention refers to a dual-guide RNA composition for executing a single-guide RNA CRISPR-associated system. The present invention gives rise to a cheaper and versatile genome editing and diagnosis platform.

## Description

### FIELD OF THE INVENTION

The present invention refers to the medical field and it is mainly related with methods and systems for genome editing and diagnosis. Particularly, the present invention refers to a dual-guide RNA composition for executing a single-guide RNA CRISPR-associated system. The present invention gives rise to a cheaper and versatile genome editing and diagnosis platform.

### STATE OF THE ART

RNA-mediated adaptive immune systems in bacteria and archaea rely on Clustered Regularly Interspaced Short Palindromic Repeat (CRISPR) genomic loci and CRISPR-associated (Cas) proteins that function together to provide protection from invading viruses and plasmids. In general, CRISPR-Cas systems fall into two classes: Class 1 systems, divided into types I, III, and IV, rely on a complex of multiple proteins to degrade foreign nucleic acids; and class 2 systems, divided into types II, V, and VI, utilize a single effector Cas protein, such as the well-studied Cas9 (a type II CRISPR system) and Cas13 (a type VI CRISPR system). The Class 2 CRISPR-Cas systems consist in a CRISPR RNA (crRNA) and a Cas protein. The crRNA contains the information to target Cas protein to a specific polynucleotide sequence. These multidomain effector proteins achieve interference by complementarity between the crRNA and the target sequence after a general recognition of a PAM (Protospacer Adjacent Motif) sequence, which is adjacent to the target DNA.

Most GE systems have been developed using Class 2 systems. The signature protein of Type I systems is Cas3, a single-stranded DNA nuclease and ATP-dependent helicase. Type III systems are characterized by Cas10, which assembles into a Cascade-like interference complex. Type IV systems have Csf1, a protein proposed to form part of a Cascade-like complex, though these systems consist of isolated Cas genes without an associated CRISPR array. Type V systems also contain a Cas9-like single nuclease, either Cpf1(Cas12a), C2c1, or C2c3. Type VI systems have Cas13 (formerly C2c2), a large protein with two HEPN (higher eukaryotes and prokaryotes nucleotide-binding) RNase domains. Type V Cas proteins have shown a variety of functions. Some can act not only on double-stranded DNA (dsDNA) but also on single-stranded DNA (ssDNA) and single-stranded RNA (ssRNA). This versatility makes the type V CRISPR-Cas system into the focus of recent studies. CRISPR/Cas systems are powerful technologies that are changing the way scientists tackle unsolved problems in basic biology, therapy and diagnosis. In its present forms, the different CRISPR/Cas systems require RNA molecules (crRNAs or sgRNAs) to direct the different Cas proteins to their DNA or RNA targets.

### Class 2, Type V CRISPR system

The first type V system identified comes from *Francisella tularensis, originally named Cpf1* (now Cas12a). There are different type V CRISPR systems that can interact with different substrate (dsDNA, ssDNA, and ssRNA). Class 2 type V is classified into 4 subtypes (V-A, V-B, V-C, V-U). Cas12a belong to the V-A subtype and possess a characteristic Ruv-C like nuclease domain related to transposons. Cas12a efficiently target spacer sequences having a 5'T-rich PAM sequence (5'-TTTN-3' or 5'-TTN-3') situated upstream of the 5'end of the non-target strand.

Collateral nuclease activity of Cas12a. Besides high-specific dsDNA cleavage, Cas12a also exhibit indiscriminate ssDNA degradation activity once is activated by its specific target through the crRNA guide. This activity degrades any available ssDNA molecule into single/double nucleotides. Unspecific ssDNA degradation presents a potential problem for therapeutic applications. However, this can be partially solved by administering sufficient concentration of crRNA molecules to revert back Cas12a to a conformation where the catalytic pocket remains inaccessible.

Several type V Cas proteins have dual-nuclease activity, such as Cas12a, Cas12c, Cas12i and Cas12j and can process pre-crRNA making them very attractive for multitarget approaches. Other worst known Cas of this family are Cas12c, Cas12d, Cas12h, Cas12i, Cas12k, and Cas12Φ.

### Class 2, Type VI CRISPR system: Cas13

As discussed before Cas9 and Cas12a have enabled many genome editing strategies. Recently, Cas13 was identified as a novel type of RNA targeting enzyme. The diverse Cas13 family contains at least four subtypes: Cas13a (C2c2), Cas13b, Cas13c, and Cas13d. Several type VI CRISPR systems have been used to generate potent genome editing tools. The best example is the CRISPR/Cas13 system which uses a guide RNA, crRNA, to bind to its target, which in this case is single-stranded RNA, instead of DNA.

Unlike Cas9 and Cas12a, Cas13 does not need a proto-adjacent motif (PAM) but some Cas13 proteins prefer an A, U or C next to the 3' protospacer flanking site (PFS), but is not essential for its activity. Cast3 proteins only bind and cut ssRNA and therefore, RNAs with secondary structures can be difficult to target. gRNAs for Cas13 must target the loops or single-stranded regions of these RNAs.

Cas13 enzymes exhibit two different catalytic activities: an RNase activity that is mediated by HEPN domains, and a guide RNA maturation activity. All Cas13 need a crRNA of about 60-66 nucleotides that allows specificity to its target. This crRNA used by Cas13 forms a short hairpin structure together with a short spacer sequence (28-30 nucleotides) that is specific for the target RNA. Once Cas13 identifies the target RNA, Cas13 cuts both this RNA (cis activity) and the nearby "collateral" RNA (trans activity). Different applications have been developed using Cas13 in mammalian cells by not detecting such collateral activity. Transcript deletion is one of the simplest *in vivo* applications of Cas13. In this case, RNA knockdown relies on the cleavage of specific transcripts by endogenous RNase activity of the two HEPN domains of the protein, the efficiency of which varies between different Cas13 orthologs and subtypes.

Cas13a function similarly to Cas9 and Cas12a, using a 64nt guide RNA (a single crRNA) to target Cas13 to the RNA target. The Cas13 protein complexes with the crRNA through a short hairpin and bind to it target through a 28 - 30-nt spacer. As described for Cas12a, Cas13 also showed collateral endonuclease activity. Indeed, in addition to specific RNase activity, all Cas13 acquire sequence-unspecific endonuclease activity after cleavage the target sequence. This activity could be part of a programmed cell death pathway in bacteria.

The collateral activity of Cas13a in mammalian cells is still unclear with conflicting reports about it. However, it is used this system for basic research and therapeutic applications. Example are transcript knockdown, live-cell transcript imaging and RNA base editing. Importantly, collateral activities of Cas12 and Cas13 can be used to cleave fluorescent reporters upon target recognition, and this has been used for the design of sensitive and specific diagnostics tools.

Different Cas13 orthologs have shown different properties in terms of RNA knockdown and editing. Also, smaller Cas13, such as Cas13d have shown to achieved efficient genome editing while been able to be packed in AAV (an important aspect for in vivo genome editing.

Another *in vivo* application of Cas13 is for RNA base editing. Dr. Zhang's group developed a programmable RNA editing system that allows modulation of different transcripts. The system, called REPAIR (RNA Editing for Programmable A to I Replacement) works by fusing the RNA-acting adenosine deaminase (ADAR2) with a catalytically inactive Cas13b (dCas13b) Cox, D.B.T., et al., RNA editing with CRISPR-Cas13.

In addition to in vivo applications, diagnostic applications have also been developed taking advantage of the collateral activity of Cas13, by being able to fragment fluorescent reporters when the target RNA is recognized. This has in fact allowed the development of the sensitive and specific diagnostic tool, called SHERLOCK (Specific High-Sensitivity Enzymatic Reporter unLOCKing) for the diagnosis of Dengue and Zika virus.

Subsequently, it has also been used to detect SARS-CoV-2 (type 2 coronavirus that causes severe acute respiratory syndrome, Severe Acute Respiratory Syndrome Coronavirus 2), the virus that causes the pandemic caused in 2019 by coronavirus (COVID-19). Subsequently, it has also been used to detect SARS-CoV-2 (type 2 coronavirus that causes severe acute respiratory syndrome, Severe Acute Respiratory Syndrome Coronavirus 2), the virus that causes the pandemic caused in 2019 by coronavirus (COVID-19)

SHERLOCK is a rapid diagnostic method that allows detection at fentomolar concentrations in a sample, making it a highly sensitive and specific method. This process includes a first step of an isothermal pre-amplification called RPA (Recombinase Polymerase Amplification), followed by a second step that combines transcription by T7 RNA polymerase, and detection by Cas13.

SHERLOCK diagnostic assays have several advantages over traditional RT-qPCR (Real Time quantitative PCR) for pathogen detection. Specifically, RT-qPCR tests that detect SARS-CoV-2 mRNA have low sensitivity, between 63% and 78%, indicating that false-positive detection is quite common compared to detection from SHERLOCK that offers greater sensitivity and specificity. Specifically, based on an early study, SHERLOCK had 100% sensitivity and 100% specificity in detecting purified Zika virus RNA ¹¹ RT-qPCR tests are labor intensive for the individual processing of each sample. In addition, they require long periods of time and high costs. Also, RT-qPCR machines can only be found in laboratory settings.

On the other hand, SHERLOCK has numerous advantages over RT-qPCR: (1) it is portable, since a lateral flow strip can be used for detection of a single target; (2) it is faster, since the entire process can be done in about 2 or 3 hours; (3) and it is also more versatile, since it can be carried out at room temperature (25 °C). Therefore, SHERLOCK detection is sensitive, specific, fast, cheap and portable, unlike RT-qPCR techniques.

In summary, there is an unmet need of finding new strategies to achieve cheaper and versatile genome editing and diagnosis platforms. The present invention is focused on solving this problem and, for that purpose, a dual-guide RNA composition, specifically designed for executing a single-guide RNA CRISPR-associated system, is herein presented.

### DESCRIPTION OF THE INVENTION

### Brief description of the invention

The present invention refers to a dual-guide RNA composition for executing a single-guide RNA CRISPR-associated system, giving rise to a cheaper and versatile genome editing and diagnosis platform. So, in other words, the dual-guide RNA composition of the invention has been specially designed to be implemented in those CRISPR-associated system which use a single-guide RNA.

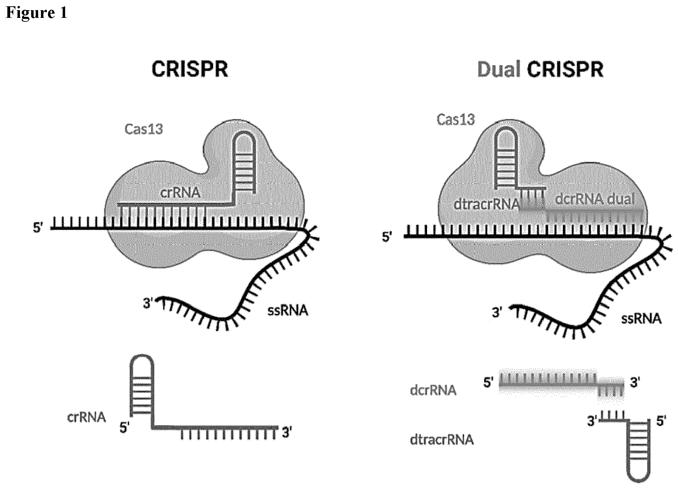

Particularly, the present invention expands the versatility of Type V and Type VI CRISPR-associated enzymes by separating the two activities of the single crRNA (protein effector binding and target binding) into two RNAs molecules each having a different activity (**Figure 1**): 1) the intermediate RNA (dtracrRNA) binds the effector protein and the guide RNA (dcrRNA) and 2) the dcrRNA binds the targeted sequence and the dtracrRNA. This dual design will increase the versatility of the systems and will reduce costs in multiplexing approaches. Dual Type V and Type VI Cas can be used to design robust and cheaper platforms for multiplexing genome editing and diagnosis. As single Type V and Type VI Cas, it can be used in living cells as well as in different fluids. Particularly, the present invention uses small dcrRNA, instead of large crRNAs, to direct the Type V and Type VI Cas proteins to their DNA or RNA targets.

So, the first embodiment of the present invention refers to a dual-guide RNA composition for executing a single-guide RNA CRISPR-associated system which comprises: a) An intermediate RNA (dtracrRNA) sequence which binds the effector protein and the guide RNA b), and b) guide RNA (dcrRNA) which binds the intermediate RNA sequence a) and the target sequence.

The second embodiment of the present invention refers to a composition or kit-of-parts which comprises: a) At least an effector protein, or a nucleic acid encoding the effector protein, and b) the above mentioned dual-guide RNA composition of the invention.

Please note that the effector proteins are usually guided to their target site by a single guide RNA (crRNA) which uniquely targets the DNA or RNA sequences to which it is complementary. This means that, by implementing the present invention, if we want to target a specific site, we have to change only the part of the spacer of de crRNA that bind the target sequence instead of engineering a whole new polynucleotide. The effector protein needs a double interaction with the DNA/RNA and the crRNA through their "stem loop" structure to start the cleaving reaction.

Particularly, according to the present invention, in order to redirect type V and Type VI Cas nucleases to new targets it is only needed to generate new 20-30bp length dcrRNAs, maintaining the same dtracrRNA for each type V/CI Cas.

In some embodiments, the CRISPR complex comprises one or more nuclear localization sequences of sufficient strength to drive accumulation of said CRISPR complex into the nucleus of eukaryotic cells.

Then, in a preferred embodiment, the composition, kit of parts or system of the invention also comprises at least a nuclear localization sequences.

In a preferred embodiment, the effector protein belongs to the type V or type VI CRISPR systems, and preferably is selected from the group consisting of: Cas12a, Cas12b, Cas12e, Cas 12c, Cas 12d, Cas 12h, Cas12i, Cas 12k, and Cas 120, Cas 12i, Cas 12j, Cas 14a, Cas13a, Cas13b, Cas13c, and Cas13d.

In a preferred embodiment, the dcrRNA comprises a target-binding region which is 15-30 nucleotides long and a dtracrRNA-binding region which is 4-12 nucleotides long.

In a preferred embodiment, the dtracrRNA comprises a dcrRNA-binding region which is 4-12 nucleotides long and an effector-protein-binding region which is 25-45 nucleotides long. In a preferred embodiment, the dtracrRNA comprises a guanine (G) as the last nucleotide (5'> 3') of the effector-protein-binding region.

In a preferred embodiment, if the effector protection is Cas13a the dtracrRNA is selected from the following sequences:
SEQ ID NO: 1
   5' GAUUUAGACUACCCCAAAAACGAAGGGGACUAAAACGAGCGC 3'
   or
SEQ ID NO: 2
   5' GAUUUAGACUACCCCAAAAACGAAGGGGACUAAAACGACAGAUA 3'
or wherein if the effector protection is Cas12 the dtracrRNA comprises the sequence SEQ ID NO: 3 5' UAAUUUCUACUCUUGUAGAU-Z' 3', wherein Z' is a polynucleotide having between 4 and 12 nucleotides that hybridizes the dcrRNA.

The third embodiment of the present invention refers to a CRISPR-associated system, preferably CRISPR-Cas system, comprising the above mentioned composition or kit-of-parts.

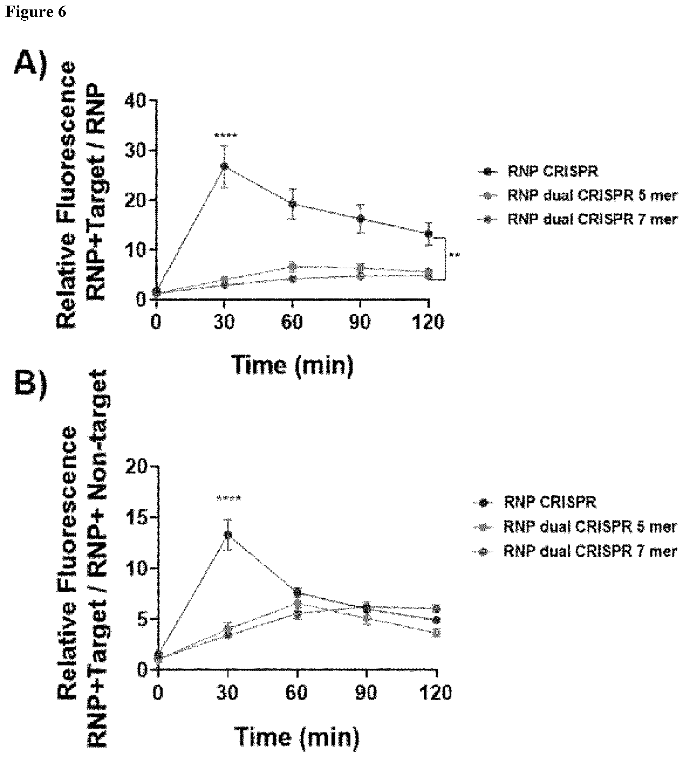

The fourth embodiment of the present invention refers to a non-viral vector comprising the above-mentioned composition or the kit of parts. The non-viral vectors may be transferred into a target cell using a non-viral system. They are preferably selected from list comprising: electroporator, liposome, polications, nanoparticles (such as lipid nanoparticles or organic and inorganic nanoparticles), cationic lipids or combinations thereof. The effector protein, the dtracrRNA and the dcrRNA can form a ribonucleopeptide complex than can act as non-viral vector (**Figure 6**).

In one embodiment of the present aspect the non-viral vector of the invention is transferred into a target cell using a non-viral system. More preferably the non-viral system is selected from the list consisting on: an electroporator, a liposome, a polication, a nanoparticle, or combinations thereof.

The fifth embodiment of the present invention refers to the above-mentioned composition or the kit of parts, CRISPR-associated system, or non-viral vector, for use as a medicament, preferably for use in the prevention, amelioration, treatment or monitoring of a disease or disorder; or in the diagnosis of a disease or disorder. Alternatively, the present invention refers to a method for the prevention, amelioration, treatment or monitoring of a disease or disorder; or in the diagnosis of a disease or disorder, which comprises the administration of the above-mentioned composition or the kit of parts, CRISPR-associated system, or non-viral vector.

The sixth embodiment of the present invention refers to the use of an intermediate RNA (dtracrRNA) sequence which binds the effector protein and the guide RNA (dcrRNA) for making a dual-guide RNA composition to execute a single-guide RNA CRISPR-associated system.

In a preferred embodiment, the present invention refers to the use of an intermediate RNA (dtracrRNA) sequence which binds the effector protein and the guide RNA (dcrRNA) to direct the effector proteins to their DNA or RNA targets in a single-guide RNA CRISPR-associated system.

In a preferred embodiment, the present invention refers to the use of a single intermediate RNA (dtracrRNA) sequence to target an effector protein to multiple target sequences through multiple guide RNAs (dcrRNAs), preferably in a single-guide RNA CRISPR-associated system. This allows e.g., elimination of multiple targets using the same effector protein, a single dtracrRNA and multiple guide RNAs (dcrRNAs) for multiple targets.

In a preferred embodiment, the present invention refers to the use of multiple intermediate RNAs (dtracrRNAs) to target multiple effector proteins to the same target sequence through a single guide RNA (dcrRNA), preferably in a single-guide RNA CRISPR-associated system. This allows multiple effector proteins to be directed to the same target. This is achieved by using the same guide RNA (dcrRNA) specific to the particular target, and multiple dtracrRNAs (sharing the same dcrRNA-binding linker) that bind to multiple different Cas.

Moreover, the present invention refers to the use of the abovementioned dual-guide RNA composition for executing a single-guide CRISPR-associated system.

Further deepening the applications of this invention, the present invention can be used for genome editing. In this regard, the present invention can be used, without limitation, to generate gene-modified cells and organisms (transgenic animals and plants). Also, it can be used to modulate gene expression of cells and organisms. For example, for increasing expression of a chromosomal sequence in a cell or embryo.

Preferably, the cell is a human cell, a non-human mammalian cell, a stem cell, a non-mammalian vertebrate cell, an invertebrate cell, a plant cell, or a single cell eukaryotic organism.

Then, another aspect of the invention refers to the composition, the kit of parts or the non-viral vectors of the invention for use in medicine.

Another aspect of the invention refers to the composition, the kit of parts or the non-viral vectors of the invention for the prevention, amelioration or treatment of a disease or disorder.

The dual CRISPR-Type V and type VI gene editing can be used to inactivate or correct gene mutations causing diseases, for example dystrophies and/or microsatellite expansion diseases, thereby providing a gene therapy approach for these groups of diseases.

Then, another aspect refers to the composition, the kit of parts or the non-viral vectors of the invention for the treatment of genetic diseases.

The guided nuclease system of the invention can target a specific region of the microorganism genome (such as bacteria, virus, parasites, fungi.). Then, the composition, the kit of parts or the non-viral vectors of the invention could be used for killing a bacterium contacting the bacterium and creating a double-stranded break in the chromosomal DNA of the bacterium. Another strategy could be making a bacterium more susceptible to an antibiotic, cleaving an antibiotic resistance gene encoded by the bacterium. Also, methods of the invention may be used to remove even latent virus genetic material from a host organism, without interfering with the integrity of the host's genetic material.

Then, another aspect refers to the composition, the kit of parts or the non-viral vectors of the invention for the treatment of infectious diseases.

The composition, the kit of parts or the non-viral vectors of the invention could be used to therapeutically target oncogene mutations or to repair defective tumor suppressor genes. That is, can be used to inactivate or correct oncogene mutations causing cancer, thereby providing a gene therapy approach for treating the underlying causes of cancer.

Also, the composition, the kit of parts or the non-viral vectors of the invention could be used to eliminate immune checkpoint genes from T cells for cancer immunotherapy approaches.

Also, the composition, the kit of parts or the non-viral vectors of the invention could be used to generated universal CAR-T cells by eliminating the TCR.

Then, another aspect of the invention relates to the composition, the kit of parts or the non-viral vectors of the invention for preventing, inhibiting, or treating cancer in a subject. For example, the guide system comprises at least one targeted genomic sequence, such as an oncogenic mutation or tumor suppressor gene.

Another aspect of the invention refers to a method, hereinafter first method of the invention, for generating specific cleavage in a double stranded DNA, in a single stranded DNA or in a single stranded RNA using the composition, the kit of parts or the non-viral vectors of the invention. More preferably said cleavage is in a cell in vitro or ex vivo. Still more preferably said cleavage is in a cell in vivo and the delivery of the ribonucleopeptide complex is performed as mentioned previously.

In another preferred embodiment, the aim of such cleavage is to modify the target sequence. In another preferred embodiment, the aim of such cleavage is to disrupt the function of a functional protein.

Another aspect of the invention refers to a method. Hereinafter second method of the invention, for specific binding to double stranded DNA, a single stranded DNA or a single stranded RNA, using the composition, the kit of parts or the non-viral vectors of the invention wherein the Cas polypeptides are mutated for their cleavage activity. More preferably said binding is in a cell in vitro or ex vivo and the delivery of the ribonucleopeptide complex is performed as mentioned previously.

In another preferred embodiment, the aim of such binding is to visualize the target sequence.

In another preferred embodiment, the aim of such binding is to detect the target sequence.

Another aspect of the invention refers to the composition, the kit of parts or the non-viral vectors of the invention for monitoring a disease or disorder.

Alternatively, the present invention can be used in the context of diagnosis. Some traditional diagnostic methods such as PCR are time consuming assays that require multiple steps and specific equipment. So, an ideal rapid diagnostic test would be sensitive and specific, easy to perform and affordable. CRISPR-based assays require minimal to no equipment and can be run as single reaction tests. They are easy to use and can deliver fast and accurate results. Dual type V and type VI CRISPR systems could also take these advantages but reducing cost. Several groups have taken advantage of the characteristics of class 2 Type V and Type IV CRISPR systems, based mainly on Cas12a and Cas13a nucleases, respectively, developing different platforms for rapid and accurate nucleic acids detection. Firstly, the Professor Doudna and her team demonstrated that combining the processing and interference activities of Cas13a it is possible the detection of cellular transcripts.

Later, the RNA-activated ssRNA-degradation activity of Cas13a was functionalized to create the SHERLOCK (Specific High Sensitivity Enzymatic Reporter UnLOCKing) platform. A paper-based assay that uses isothermal amplification and Cas13a for *in vitro* detection of specific strains of Dengue and Zika virus with attomolar sensitivity, distinguishes pathogenic bacteria, identifies low frequency DNA mutations (e.g., SNP) correlated with cancer and other diseases, or human genotyping. Combination of orthogonal CRISPR enzymes, which present discrete crRNA and substrate, and other advances, promoted a more advanced SHERLOCK platform, known as SHERLOCKv2, which allows simultaneously detection of Zika and Dengue virus, and mutations in liquid biopsy samples. Similarly, exploiting the multiple-turnover nuclease activity of Cast2a Chen et al have developed DETECTR (DNA endonuclease-targeted CRISPR trans reporter) 16, a method that with attomolar sensitivity allows specific nucleic acid detection between two types of human papillomavirus (HPV). This platform has been applied for detection of beta-coronavirus severe acute respiratory syndrome (SARS)-CoV-2 (COVID-19) from respiratory swab RNA extracts in a portable, easy to perform, rapid and accurate manner.

Within this context, SHERLOCK has been used to detect ctDNA sequences with high sensitivity and specificity following their isolation, amplification via RPA and conversion to single strand RNA through the use of a T7 RNA polymerase. In this way, Zhang Group reported the detection of cancer-related EGFR-L858R and BRAFV600E genomic mutations in mock ctDNA samples containing as low as 0.1% mutant alleles 15. In another study, the SHERLOCK method was successfully applied for the detection of mutant alleles. Liquid biopsy in non-small-cell lung cancer (NSCLC) patients was performed and detected EGFR-L858R, EGFR-T790M, and EGFR exon 19 deletion mutations. Overall, the CRISPR-Cas13 system is a highly sensitive and specific tool for ctDNA detection from liquid biopsy samples.

The RAS family of genes and proteins is one of the most commonly altered drivers in cancer. Mutations in codons 12 and 13 of exon 2 result in a conformational change in the KRAS protein. As a consequence, its dephosphorylation is inhibited, leading to constitutive activation and thus making therapeutic interventions targeting EGFR (its upstream regulator), ineffective. For these reasons, KRAS genotyping of tumour tissues are required to be performed before a monoclonal antibody therapy is prescribed.

Another aspect of the invention refers to the diagnosis of an infectious disease by detecting specific regions of the pathogen nucleic acids.

Then, another aspect of the invention refers to the diagnosis of a disorder by detecting DNA/RNA single point mutations, indel or alternative splicing variants.

For the purpose of the present invention the following terms are defined:
- In the context of the present invention, the "guide system" of the invention comprising the dtracrRNA sequence and the dcrRNA, is equivalent at the crRNA of the type V and type VI Cas systems. The dtracrRNA keep the direct repeat `DR' domain binding Cas proteins and the dcrRNA keep the target specificity (spacer).
- The "kit of parts" or "system" of the invention refers to a combination of a set of components suitable for targeting specific DNA or RNA sequences which may or may not be administered together. The components of the kit (the CRISPR enzyme - Cas polynucleotide- and the guide system) can be provided in separate vials (in the form of "kit of parts") or in a single vial. On the other hand, the parts of the kit of the invention can be jointly or separately sold/administered. In this regard, it should be emphasized that the term "kit of parts" means that the components of the system of the invention (effector protein, dtracrRNA, dcrRNA) do not need to be present in the same composition, in order to be available for their combined, separate or sequential application. Thus, the expression "kit of parts" implies that a true combination does not necessarily result, in view of the physical separation of the components.
- Unless otherwise specified, "a," "an," "the," and "at least one" are used interchangeably and mean one or more than one.
- As used herein, "nucleic acid" or "polynucleotides" refers to nucleotides and/or polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally occurring nucleotides (such as DNA and RNA), or analogues of naturally occurring nucleotides (e.g., enantiomeric forms of naturally occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Nucleic acids can be either single stranded or double stranded.
- The terms "vector" or "vectors" refer to system capable of transporting the desire molecule, or combination of molecules into the target cell. A "vector" in the present invention includes, but is not limited to, a viral vector, and a non-viral vector such as plasmid, a linear RNA or DNA, and ribonucleoprotein (RNP) complexes. RNA and DNA may consist of a chromosomal, non-chromosomal, semi-synthetic or synthetic nucleic acid. Large numbers of suitable vectors are known to those of skill in the art and commercially available.
- Delivery vectors and vectors can be associated or combined with any cellular permeabilization techniques such as sonoporation or electroporation or derivatives of these techniques.
- By "mutation" is intended the substitution, deletion, insertion of up to one, two, three, four, five, six, seven, eight, nine, ten, eleven, twelve, thirteen, fourteen, fifteen, twenty, twenty-five, thirty, forty, fifty, or more nucleotides/amino acids in a polynucleotide (cDNA, gene) or a polypeptide sequence. The mutation can affect the coding sequence of a gene or its regulatory sequence. It may also affect the structure of the genomic sequence or the structure/stability of the encoded mRNA.
- By "variant(s)", it is intended a repeat variant, a variant, a DNA binding variant, a TALE-nuclease variant, a polypeptide variant obtained by mutation or replacement of at least one residue in the amino acid sequence of the parent molecule.
- By "functional variant" is intended a catalytically active mutant of a protein or a protein domain; such mutant may have the same activity compared to its parent protein or protein domain or additional properties, or higher or lower activity.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default setting. For example, polypeptides having at least 70%, 85%, 90%, 95%, 98% or 99% identity to specific polypeptides described herein and preferably exhibiting substantially the same functions, as well as polynucleotide encoding such polypeptides, are contemplated.
- "Similarity" describes the relationship between the amino acid sequences of two or more polypeptides. BLASTP may also be used to identify an amino acid sequence having at least 70%, 75%, 80%, 85%, 87.5%, 90%, 92.5%, 95%, 97.5%, 98%, 99% sequence similarity to a reference amino acid sequence using a similarity matrix such as BLOSUM45, BLOSUM62 orBLOSUM80. Unless otherwise indicated a similarity score will be based on use of BLOSUM62. When BLASTP is used, the percent similarity is based on the BLASTP positives score and the percent sequence identity is based on the BLASTP identities score. BLASTP "Identities" show the number and fraction of total residues in the high scoring sequence pairs which are identical; and BLASTP "Positives" show the number and fraction of residues for which the alignment scores have positive values, and which are similar to each other. Amino acid sequences having these degrees of identity or similarity or any intermediate degree of identity of similarity to the amino acid sequences disclosed herein are contemplated and encompassed by this disclosure. The polynucleotide sequences of similar polypeptides are deduced using the genetic code and may be obtained by conventional means. A polynucleotide encoding such a functional variant would be produced by reverse translating its amino acid sequence using the genetic code.
- The term "subject" or "patient" as used herein includes all members of the animal kingdom including non-human primates and humans.
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of'. Thus, the phrase "consisting of" indicates that the listed elements are required or mandatory, and that no other elements may be present.

### Brief description of the figures

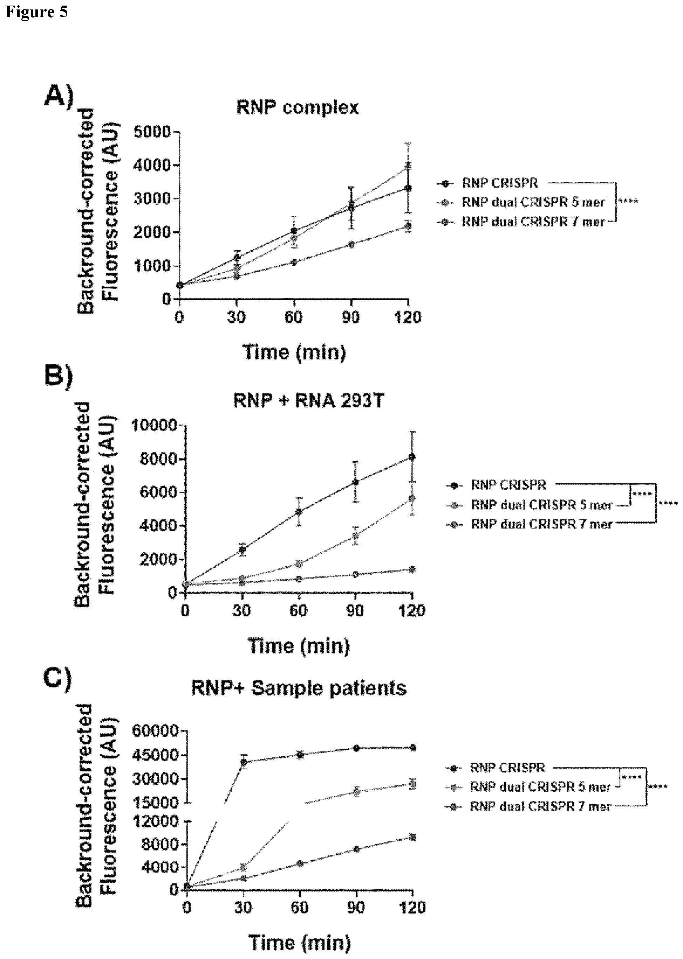

**Figure 1****. Schematic design of dual-CRISPR type V and type VI systems.** The structures of the CRISPR (left) and dual CRISPR (right) systems are shown. In CRISPR, (Cas13 as example), Cas binds to a single crRNA, which also serves as a junction between the target sequence and Cas. To develop a dual CRISPR design from this system, we have dissociated the single crRNA into two RNA molecules, one harboring the target-binding function (dcrRNA) and the other, the Cas-binding function (dtracrRNA), and each one binding each other. The final design will have three components: the protein Cas13, the variable dcrRNA, that will bind to the chosen target sequence and the dtracrRNA, designed for binding the desired type V or type VI Cas protein (in this case, Cas13) as well as the dcrRNA. Figure created with BioRender^{©}.

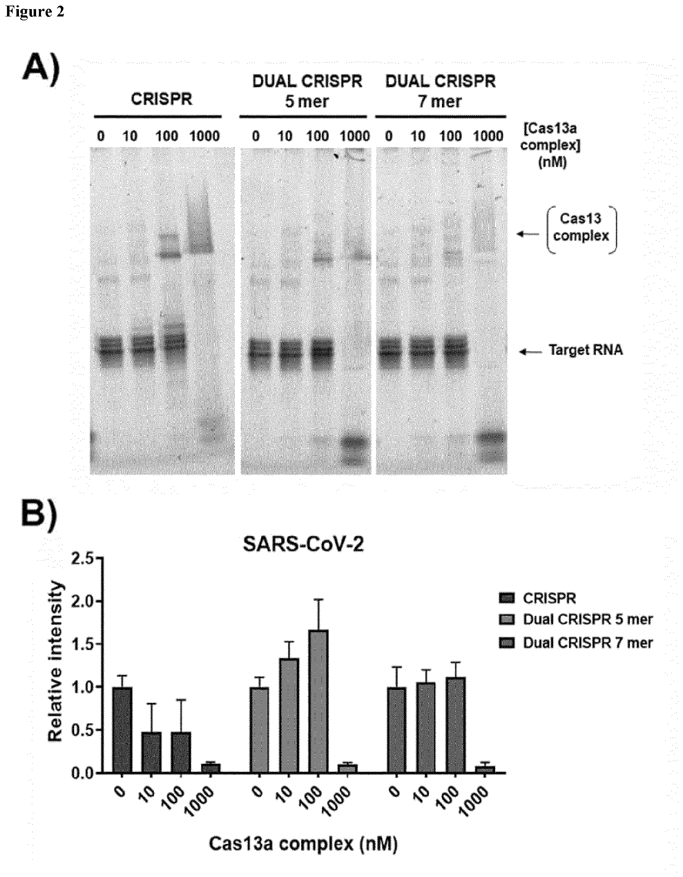

**Figure 2****. Type V Cas (Cas13a) bind to its target through the dual system: dcrRNA/dtracRNA.** A) Electrophoretic mobility shift assay (EMSA) in CRISPR, dual CRISPR (5 mer) and dual CRISPR (7mer) for the SARS-CoV-2 RNA target. The ssRNA (200 ng) was incubated with increasing concentrations (0, 10, 100 and 1000 nM) of the Cas13a-sgRNA/dtracrRNA:dcrRNA complex reconstituted in vitro. The lower and upper bands represent the fractions of DNA unbound and bound to the complex, respectively. B) Graph of the relative binding intensity of the CRISPR and dual CRISPRs (5 and 7 mer) to their target RNA. In each case, it has been relativized against RNA without complex (0 nM).

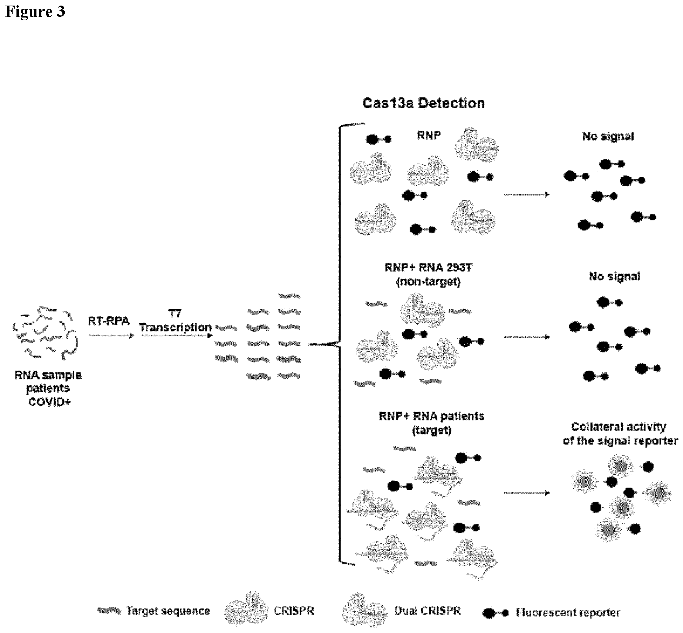

**Figure 3****. SHERLOCK screening assay.** Diagram of the main steps of the SHERLOCK assay. The process begins with the pre-amplification of a sample of either RNA or DNA that has the target sequence to be detected. The amplified fragments are converted to RNA through T7 transcription and then detected by CRISPR or dual CRISPR complexes, which are activated by recognizing their target and begin to fragment RNA fluorescent reporters. Figure adapted from Kellner et al.

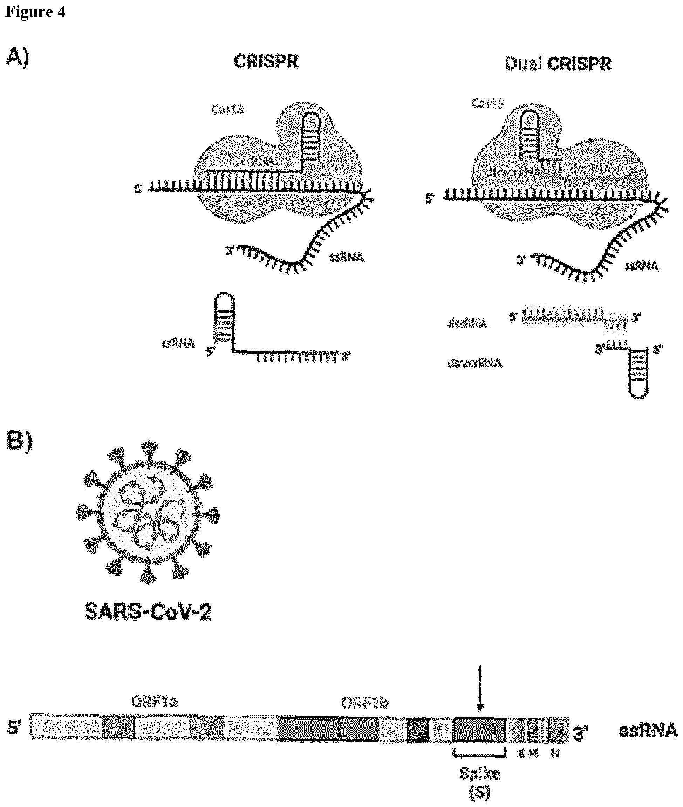

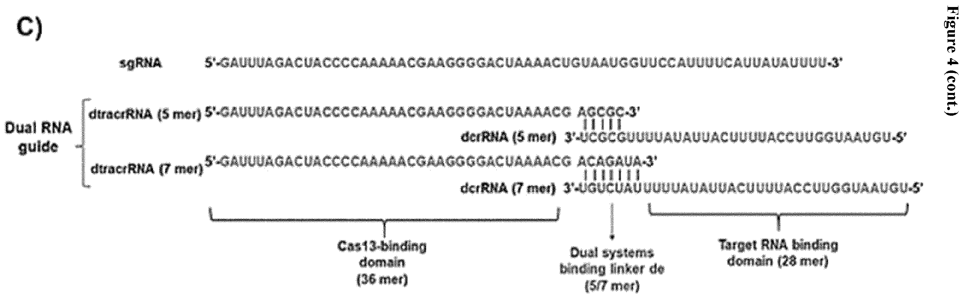

**Figure 4****. Design of the CRISPR and CRISPR dual for the detection of SARS-Cov2using SHERLOCK. A)** Schematic of the CRISPR dual design for CRISPR Type VI (Cas13) systems. The structures of the CRISPR (left) and dual CRISPR (right) systems are shown. In CRISPR, Cas13 binds to crRNA, which serves as a junction between the target sequence and Cas. The dual system has been generated by dissociating the DNA-binding (dcrRNA) and Cas-binding (dtracrRNA) functions. The final design will have three components: the Cas13 protein, the variable dcrRNA, that will bind to the chosen target sequence, and the dtracrRNA designed for Cas13, which will bind both Cas13 and crRNA dual. Figure created with BioRender^{©}. **B)** Scheme of the genome of the SARS-CoV2 virus genome to which we will direct our systems. Specifically, the designs of the different guides, whether they are sgRNA or dcrRNA, will be directed to the *s* gene that encodes the S protein. Created with BioRender^{©}. **C)** Specific sequences of the different components for the CRISPR (top) and dual CRISPR (bottom) systems for LwCas13a. The LwCas13a binding domain is marked in green, and the 28-nucleotide spacer domain that recognizes the ssRNA of the *s* gene is marked in blue, for sgRNA (CRISPR) and crRNA (dual CRISPR). In orange, the linkers that join the dual systems (5 and 7 mer) are shown. In red, a guanine (G) appears in the tracrRNA, which is a constant area to avoid steric interactions in the LwCas13a pocket. The sequence of Figure 4 c) are as follows:
   - SEQ ID NO: 12 sgRNA: 
GAUUUAGACUACCCCAAAAACGAAGGGGACUAAAACUGUAAUGGUUCCAUUU
UCAUUAUAUUUU
   - SEQ ID NO: 13 dtracrRNA (5 mer):
      GAUUUAGACUACCCCAAAAACGAAGGGGACUAAAACGAGCGC
   - SEQ ID NO: 14 dcrRNA (5 mer)
      UCGCGUUUUAUAUUACUUUUACCUUGGUAAUGU
   - SEQ ID NO: 15 dtracrRNA (7 mer):
      GAUUUAGACUACCCCAAAAACGAAGGGGACUAAAACGACAGAUA
   - SEQ ID NO: 16 dcrRNA (7 mer)
      UGUCUAUUUUUAUAUUACUUUUACCUUGGUAAUGU
**Figure 5****. Dual Cas13a maintain endonuclease specificity and collateral activity of Cas13**. Collateral endonuclease activity using Sherlock assay of CRISPR/Cas13 (green), dual CRISPR/Cas13-5mer (red) and dual CRISPR/Cas13-7mer (blue) in the absence of any ARN (**A**), in the presence of control RNA (**B**) and in the presence of targeted RNA from COVID patients (**C**). Statistical analysis: 2-way ANOVA (Dunnett test multiple comparison of mean fluorescence at all times (A-C) (* p < 0.05, ** p < 0.01, *** p < 0.001, **** p < 0.001). The values represent the mean ± SEM of at least 4 separate experiments.
**Figure 6****. Evaluation of the specificity of SARS-Cov-2 detection of the dual CRISPR/Cas13 complexes versus standard CRISPR/Cas13. A) Specificity in the absence of untargeted (control) RNA.** Graph shows the ratio of the fluorescence obtained by each RNP complex in the presence of patient samples (target RNA) divided by the fluorescence of RNP complexes alone (only fluorescence probe added). **B**) **Specificity** in the **presence of control RNA.** Graph shows the ratio of the fluorescence obtained by each RNP complex incubated with patient samples (target RNA) divided by the fluorescence of each RNP complex incubated in the presence of non-target RNA (293T). Statistical analysis: 2-way ANOVA (Dunnett's multiple comparison test at 30 and 330 min) (* p < 0.05, ** p < 0.01, *** p < 0.001, **** p < 0.001). Values represent the mean ± SEM of at least 4 separate experiments.

### Detailed description of the invention

The present invention is illustrated by means of the Examples set below, without the intention of limiting its scope of protection.

### EXAMPLE 1. Design of the dual type V and type VI CRISPR system of the invention

In general terms, the dual-guide RNA composition of the invention has been generated following the criteria below:
- dtracrRNA design: The type V or type VI Cas-binding area (forming a stem-loop, also named direct repeat or `DR') is underlined and is 25-45 nucleotides long. The dcrRNA binding area (complementary to area in dcrRNA highlighted in bold letters) is represented in bold letters and is 4-12 nucleotides long.
   (5' XXXXXXXXXXXXXXXXXXXXXXXXXXXXXXX**GAX'X'X'X'X** 3')
- dcrRNA design: The target-binding area is underlined and is 15-30 nucleotides long. The tracrRNA binding region represented in bold letters and is 4-12 nucleotides long.
   (5' XXXXXXXXXXXXXXXXXXXXXXXXXXXXX**XXXXXX** 3')

The design of the dual type V and type VI CRISPR systems are based on the replacement of the single crRNA spacer by two RNA molecules: dtracrRNA and dcrRNA. Since different type V and type VI CRISPR systems have different domains in the crRNA for binding the different Cas, the dual type V and type VI CRISPR systems designs will differ in the underlined region of dtracrRNA but can share the dcrRNA binding domain. Therefore, with the dual platform of the present invention, it could be possible to use the same dcrRNA to target different type V/VI Cas proteins if their dtracrRNA share the same domain represented in bold letters.

To design a dual CRISPR Cas13a system (**Figure 2**), the dtracrRNA must have de `DR' domain that bind Cas13a. Two alternative dtracrRNA for Cas13a have been designed:
**dtracrRNA-13a-5mer:** 5' GAUUUAGACUACCCCAAAAACGAAGGGGACUAAAAC**GAGCGC** 3' (SEQ ID NO: 1)
**dtracrRNA-13a-7mer:** 5' GAUUUAGACUACCCCAAAAACGAAGGGGACUAAAAC**GACAGAUA** 3' (SEQ ID NO: 2)
where the Cas13a-binding area ('DR' domain) is represented in underlined and the dcrRNA-binding area is shown in represented in bold letters. Dual guide RNAs (dcrRNAs) will be designed, having a region complementary to the target sequence and a 5 or 7 nucleotide sequence complementary to the dtracrRNA-13a-5mer or dtracrRNA-13a-7mer, respectively. Additionally, a constant zone (G) was included to avoid steric interactions between the pocket of LwCas13a and the 5-7mer sequences.

Firstly, we analyzed whether the dual CRI SPR/C as 13 a system could make RNP complexes able to bind specific RNA targets (**Figure 3**). An EMSA was conducted to analyze efficiency of RNP-complex formation and the ability to bind and degrade its RNA target. The target ssRNA was incubated with increasing concentrations (0, 10, 100 and 1000 nM) of the CRISPR complex (Cas13a-crRNA) (**Figure 3A**, left panel) or the dual CRISPR complex (Cas13-dcrRNA-dtracrRNA of 5 mer and 7 mer) (**Figure 3A**, middle and right panels respectively) complexes formed in vitro. The results showed how the target RNA band disappeared as the complex concentration increased (**Figure 3A** and **3B**). These data indicate that, like the dual-CRISPR/Cas 13 system as the original CRISPR/Cas13 can recognize target RNA and degrade it due to its cis RNase activity.

### Example 2. Applications of dual CRISPR system of the invention

Since type V/VI dual-CRISPR can maintain similar nuclease activity compared to the original CRISPR systems to which they derived **(****Figure 3****),** the potential applications of this platform are similar: DNA and RNA genome editing for basic research, biotechnology, industry, therapeutic and diagnostic applications.

### Example 2.1. Diagnostic examples using the dual type V/VI CRISPR system of the invention

Several biomarkers can be used for diagnostic, but nucleic acid (NA) is the gold standard for a wide array of diseases (particularly those caused by infectious agents) due to it sensitivity and specificity. Besides human disease monitoring, NA detection is also employed for food safety and agriculture. However, NA testing platforms are expensive, require scientific instrumentation and must be confined in laboratories. CRISPR-based diagnostics platforms have emerged as a promising alternative. They are inexpensive, simple, and do not require the use of special instrumentation.

### Example 2.1.1. Detection of SARS-COV2 virus

We have developed a tool for diagnostic of SARS-Cov2 based on the dual Cas13 system (**Figure 2**) Samples with SARS-Cov2 RNA are treated with reverse-transcriptase-recombinase polymerase amplification (RT-RPA) to amplify the *s* gene fragment. Then, an in vitro T7 transcription step take place before putting in contact with Cas13 complex formed by dcrRNA and dtracrRNA. In one example, a fluorescent reporter (reporter 1) is used. A fluorescent plate-reader is used to detect the SARS-Cov-2.

SHERLOCK analyses (**Figure 5** **and** **6**) were performed with the CRISPR and dual CRISPR (5 and 7 mer) RNP complexes. Since this analysis require the collateral activity of Cas13, we first investigated whether the dual CRISPR system maintained this RNase activity. To do this, samples with COVID + patients (provided by the company Lorgen (Granada, Spain)), were used to perform SHERLOCK in two steps.

The first step consisted of an RT-RPA amplification. The second step was to synthesize again RNA with T7 and incubated with the different RNP complexes. Importantly, we observed collateral activity in dual-CRISPR/Cas13a only in the presence of target RNA as detected by a fluorescent RNA reporter (**Figure 5C**). To compare the specificity and efficacy of detection, two controls were used. The first control was to incubate the different complexes with reporter fluorescent RNA, but without target RNA (**Figure 5A**). In this case, no signal should be obtained in any case, since there is no union of Cas13 with its target, the collateral activity of Cas13 should not be activated. Interestingly, both dual RNP complexes presented lower residual collateral RNase activity in the absence of target RNA. This is an important fact, because it could indicate that the dual CRISPR/Cas13 system has better control of trans RNase activity than the CRISPR system.

The other control included RNA from HEK293 as non-targeted RNA, in addition to the fluorescent reporter. As before, the original CRISPR/Cas13 presented the higher collateral activity in the presence of target RNA, followed very closely by the dual system of 5 mer (**Figure 5B**). Interestingly, the dual system of 7 mer presented a very low nuclease activity in the absence of target ARN. All these results show that, under our conditions, there is a nonspecific nuclease activity of the original CRISPR/Cas13 complex in the absence of RNA that is increased in the presence of non-target RNA. Interestingly, the dual CRSIPR/Cas13 7mer system maintained very low RNase activity under these conditions.

We finally incubated the different complexes with different target RNAs from patients with COVID (**Figure 5C**). Unlike in the control cases, the dual CRISRNA/Cas13 7 mer degraded fluorescent reporter RNA, although less efficiently than the original CRISPR/Cas13 and the dual CRISPR/Cas13 5mer. This result demonstrates that both dual complexes CRISRNA/Cas13 contain collateral RNase activity that is specifically activated in the presence of target RNA. As expected, the original CRISPR/Cas13 system contained the greatest collateral activity (saturated at 30 minutes), closely followed by the 5 mer dual CRISPR/Cas13 complex.

To determine the degree of specificity of the different complexes, we proceeded to correct the activity detected in the presence of target RNA (**Figure 5C**) with that detected in each of the controls (**Figure 5A and 5B**): **Figure 6A** shows the relativization against the fluorescence signal shown by the different complexes alone. In this case, after 30 minutes, it is observed that the specificity of the traditional CRISPR complex is much higher than the other complexes, since the specific signal relative to the nonspecific signal reaches the maximum. However, as time goes by, this difference is reduced, as the background of RNP CRISPR alone increases, reaching levels very similar to those obtained by the CRISPNA/Cas13 systems, as well as dual CRISPR/Cas13 of 5 mer (only significant differences were observed with the dual of 7 mer).

Perhaps more relevant is **Figure 6B****,** where the specific fluorescence signal (target RNA from COVID patients) is relativized against the fluorescence signal of the complexes in the presence of non-target RNA (293T). As in the previous case, at 30 minutes, the specificity of the traditional CRISPR/Cas13 complex is the best. However, as time goes on, the CRISPR/Cas13 system reaches the same levels as the dual CRISPR/Cas13 systems.

In another example, a lateral flow system could be used, so that when a reporter is cleaved (reporter 2) the presence of SARS-Cov2 could be identified.

### Example 2.1.2. Detection of KRAS mutations in colorectal cancer

In another example, the invention is used to detect DNA of the wild type sequence of KRAS, KRAS-G12D, KRAS-G12V and KRAS-G12C mutations located at codon 12 of exon 2 of KRAS. List of sequences are found below:
- KRAS-wt
   SEQ ID NO: 4 
FW:TAAGGCCTGCTGAAAATGACTGAATATAAACTTGTGGTAGTTGGAGCTGGTG
GCGTAGGCAAGAGTGCCTTGACGATACAGCTAA
- KRAS-G12C
   SEQ ID NO: 5 
FW:TAAGGCCTGCTGAAAATGACTGAATATAAACTTGTGGTAGTTGGAGCTTGTG
GCGTAGGCAAGAGTGCCTTGACGATACAGCTAA
- KRAS-G12D
   SEQ ID NO: 6 
FW:TAAGGCCTGCTGAAAATGACTGAATATAAACTTGTGGTAGTTGGAGCTGATG
GCGTAGGCAAGAGTGCCTTGACGATACAGCTAA
- KRAS-G12V
   SEQ ID NO: 7

FW:TAAGGCCTGCTGAAAATGACTGAATATAAACTTGTGGTAGTTGGAGCTGTTG
GCGTAGGCAAGAGTGCCTTGACGATACAGCTAA

dcrRNA sequences complementary to each of the KRAS variants is incubated with the dtracrRNA sequence and C as 13 protein to form the RNP complex.

The four RNP complexes plus a fluorescent reporter are then put in contact in individual wells with target ssRNA sequences obtained from DNA following an amplification step via RPA and final conversion to the target ssRNA using a T7 polymerase. The well-plate is then inserted in a fluorescence plate reader and, as above, fluorescence signals for each well are monitored over time. Wild-type samples are used as internal controls. For each sample, then 4 wells are run. To note, that as KRAS mutations are always heterozygotic the well with the wild-type RNP will always produce a fluorescence signal.

In another example, the invention could be used to detect RNA of the wild type sequence of KRAS, KRAS-G12D, KRAS-G12V and KRAS-G12C mutations located at codon 12 of exon 2 of KRAS. List of sequences are found below:
- KRAS-wt
   SEQ ID NO: 8 
UAAGGCCUGCUGAAAAUGACUGAAUAUAAACUUGUGGUAGUUGGAGCUGGUG
GCGUAGGCAAGAGUGCCUUGACGAUACAGCUAA
- KRAS-G12C
   SEQ ID NO: 9 
UAAGGCCUGCUGAAAAUGACUGAAUAUAAACUUGUGGUAGUUGGAGCUUGUG
GCGUAGGCAAGAGUGCCUUGACGAUACAGCUAA
- KRAS-G12D
   SEQ ID NO: 10 
UAAGGCCUGCUGAAAAUGACUGAAUAUAAACUUGUGGUAGUUGGAGCUGAUG
GCGUAGGCAAGAGUGCCUUGACGAUACAGCUAA
- KRAS-G12V
   SEQ ID NO: 11

UAAGGCCUGCUGAAAAUGACUGAAUAUAAACUUGUGGUAGUUGGAGCUGUUG
GCGUAGGCAAGAGUGCCUUGACGAUACAGCUAA

The four RNP complexes plus a fluorescent reporter are then put in contact in individual wells with target ssRNA sequences obtained from RNA following an amplification step via RT-RPA and final conversion to the target ssRNA using a T7 polymerase. The well-plate is then inserted in a fluorescence plate reader and, as above, fluorescence signals for each well are monitored over time. Wild-type samples are used as internal controls. For each sample, then 4 wells are run. To note, that as KRAS mutations are always heterozygotic the well with the wild-type RNP will always produce a fluorescence signal.

### Example 3. Genome edition examples using dual typeV/VI CRISPR systems of the invention

CRISPR technology based on Type II Cas9 systems has been used to develop new therapeutic strategies (Gene Therapy), to engineer stem cells, generate animal models, and to develop transgenic animals and plants that are resistant to diseases or severe conditions or have improved nutritionals values. Similarly, type V and type VI CRISPR systems have potential applications in all these fields. However different type V and type VI CRISPR systems are more suitable for diagnostics that type II due to their collateral activity. Also, this collateral activity can hinder their applications of some of these systems for therapeutic applications.

### Example 3.1. Generating temporal multiple gene KO T cells using dual CRISPR/Cas 13 system of the invention

Primary human T cells (isolated from Apheresis products from healthy donors and activated for 48h), will be nucleofected with single CRISPR/Cas13 or dual CRISPR/Cas13 RNPs designed to eliminate in PD1, LAG3, TIM3, Foxp3 mRNA to generate more potent-effector T cells during T-cells expansion. This platform can be used to:
- Increase tumor infiltrated lymphocytes (TILs) efficacy temporally.
- To generate CAR-T cells that are more potent for a certain period, avoiding potential, life threatening side effects.

## Claims

1. A dual-guide RNA composition for executing a single-guide RNA CRISPR-associated system which comprises:
a. An intermediate RNA (dtracrRNA) sequence which binds the effector protein and the guide RNA (b), and
b. Guide RNA (dcrRNA) which binds the intermediate RNA sequence (a) and the target sequence.

2. Composition or kit-of-parts which comprises:
a. At least an effector protein, or a nucleic acid encoding the effector protein, and
b. The dual-guide RNA composition of claim 1.

3. Composition or kit-of-parts, according to claim 2, wherein the effector protein belongs to the type V or type VI CRISPR systems, and preferably is selected from the group consisting of: Cas12a, Cas12b, Cas12e, Cas12c, Cas12d, Cas12h, Cas12i, Cast 2k, and Cas12Ø, Cas12i, Cas12j, Cas14a, Cas13a, Cas13b, Cast 3 c, and Cas13d.

4. Composition or kit-of-parts, according to any of the claims 2 or 3, wherein the dcrRNA comprises a target-binding region which is 15-30 nucleotides long and a dtracrRNA-binding region which is 4-12 nucleotides long.

5. Composition or kit-of-parts, according to any of the claims 2 to 4, wherein the dtracrRNA comprises a dcrRNA-binding region which is 4-12 nucleotides long and an effector-protein-binding region which is 25-45 nucleotides long.

6. Composition or kit-of-parts, according to any of the claims 2 to 5, wherein the dtracrRNA comprises a guanine (G) as the last nucleotide (5'> 3') of the effector-protein-binding region.

7. Composition or kit-of-parts, according to any of the claims 2 to 6, wherein if the effector protection is Cas13a the dtracrRNA is selected from the following sequences: SEQ ID NO: 1 or SEQ ID NO: 2; or wherein if the effector protection is Cas12 the dtracrRNA comprises the sequence SEQ ID NO: 3-Z wherein Z' is a polynucleotide having between 4 and 12 nucleotides that hybridizes the dcrRNA.

8. CRISPR-associated system, preferably CRISPR-Cas system, comprising the composition or kit-of-parts of claims 2 to 7.

9. Non-viral vector comprising the composition or the kit of parts of claims 2 to 7.

10. The composition or the kit of parts of claims 2 to 7, CRISPR-associated system of claim 8, or non-viral vector of claim 9, for use as a medicament, preferably for use in the prevention, amelioration, treatment or monitoring of a disease or disorder, or in the diagnosis of a disease or disorder.

11. Use of an intermediate RNA (dtracrRNA) sequence which binds the effector protein and the guide RNA (dcrRNA) for making a dual-guide RNA composition to execute a single-guide RNA CRISPR-associated system.

12. Use, according to claim 11, of an intermediate RNA (dtracrRNA) sequence which binds the effector protein and the guide RNA (dcrRNA) to direct the effector proteins to their DNA or RNA targets.

13. Use, according to claims 11 or 12, of a single intermediate RNA (dtracrRNA) sequence to target an effector protein to multiple target sequences through multiple guide RNAs (dcrRNAs).

14. Use according to claims 11 or 12, of multiple intermediate RNAs (dtracrRNAs) to target multiple effector proteins to the same target sequence through a single guide RNA (dcrRNA).

15. Use of the dual-guide RNA composition of claim 1 for executing a single-guide CRISPR-associated system.
